# EUROPÄISCHE PATENTSCHRIFT

(11) **EP 0 640 071 B1**
(45) Veröffentlichungstag und Bekanntmachung des Hinweises auf die Patenterteilung: **24.03.1999**
(21) Anmeldenummer: 93909901.6
(22) Anmeldetag: 06.05.1993
(51) Int. Cl.: C07C 263/18, C07D 251/34, C08G 18/70, C08G 18/10

(54) **VERWENDUNG VON FLÜSSIGEN ESTERN ALS LÖSUNGSMITTEL FÜR ISOCYANATE**
USE OF LIQUID ESTERS AS SOLVENTS FOR ISOCYANATES
UTILISATION D'ESTERS LIQUIDES COMME SOLVANTS POUR DES ISOCYANATES

(30) Priorität: 14.05.1992 DE 4215873
(43) Veröffentlichungstag der Anmeldung: 01.03.1995
(73) Patentinhaber: Henkel Kommanditgesellschaft auf Aktien, 40191 Düsseldorf (DE)
(72) Erfinder: KLEIN, Johann, D-4000 Düsseldorf 1 (DE); DAUTE, Peter, D-4300 Essen 1 (DE); GRÜTZMACHER, Roland, D-5603 Wülfrath (DE); HÖFER, Rainer, D-4000 Düsseldorf 30 (DE)
(86) Internationale Anmeldenummer: EP9301104
(87) Internationale Veröffentlichungsnummer: WO9323367

(56) Entgegenhaltungen:
- EP-A- 0 456 062
- US-A- 4 067 834

## Beschreibung

Die Erfindung betrifft die Verwendung von Estern als Lösungsmittel für Isocyanate und/oder Isocyanurate.

Isocyanate im Rahmen der Erfindung sind sowohl klassische Isocyanate, die an aromatischen, aliphatischen oder cycloaliphatischen Kohlenwasserstoffen NCO-Gruppen tragen als auch Prepolymere, die auch nach Umsetzung der klassischen Isocyanate mit Polyolen freie NCO-Gruppen tragen.

Insbesondere die mehrfunktionellen Isocyanate mit 2 oder mehr NCO-Gruppen sind hochviskose Produkte. Die hohe Viskosität dieser Produkte bedingt häufig Schwierigkeiten bei den Verarbeitungsschritten der Isocyanate zu Polyurethanen, da die hochviskosen Isocyanate nur schwer pumpbar und mit weiteren Komponenten nur schlecht mischbar sind. Daher setzt man den Isocyanaten Lösungsmittel zu, die die Viskositäten minimieren. Auch die trimeren Isocyanurate, die man bei der Herstellung von Polyurethanen häufig zusetzt, sind hochviskose Produkte, deren Viskosität man durch Lösungsmittel zu reduzieren versucht.

Als Lösungsmittel werden bis heute meist organische Lösungsmittel vom Typ der halogenierten Kohlenwasserstoffe wie Methylenchlorid, 1,2-Dichlorpropan, Aromaten wie Toluol, Ether wie Petrolether oder auch dünnflüssige Phosphate wie Trioctylphosphat zugesetzt, wobei letzteres gleichzeitig weichmachende Wirkung hat. Aus ökologischen und toxikologischen Überlegungen heraus, möchte man aber zunehmend auf die bedenklichen bisherigen Lösungsmittel verzichten und diese durch ökologisch und toxikologisch unbedenklichere Stoffe ersetzen.

Aus der amerikanischen Patentschrift US 2 801 244 ist es bekannt, daß sich die trifunktionellen Isocyanattrimere von 4-Alkyl-m-phenylendiamindiisocyanaten mit niederen Alkylgruppen auch in Ethylacetat lösen lassen. Ethylacetat ist aber ein sehr flüchtiger Ester, der schon unter den Verarbeitungsbedingungen zum Teil entweicht. Aus US-A-4 067 834 ist bekannt, daß sich spezielle Ester als Modifizierungsmittel bei Organometall-katalysierten Umsetzungen von Dihydroxyverbindungen mit Diisocyanaten einer Funktionalität von wenigstens 2,5 eignen.

Aufgabe der vorliegenden Erfindung war es, Lösungsmittel für Isocyanate und Isocyanurate bereit zu stellen, die toxikologisch und ökologisch nicht bedenklich sind und eine höhere Flüchtigkeit als Ethylacetat aufweisen.

Überraschenderweise können spezielle Ester die Viskositäten der Isocyanate und Isocyanurate bereits bei geringsten Zugabemengen drastisch reduzieren.

Gegenstand der vorliegenden Erfindung ist daher die Verwendung von bei Raumtemperatur flüssigen Estern von Fettsäuren mit 6 bis 22 C-Atomen mit monofunktionellen Alkoholen als Lösungsmittel für Isocyanate und/oder Isocyanurate.

Die erfindungsgemäß zu verwendenden Ester sind Ester von Fettsäuren mit 6 bis 22 C-Atomen mit monofunktionellen Alkoholen. Im Sinne der Erfindung können sowohl die Fettsäuren als auch die Alkohole gesättigt und/oder ungesättigt sein. Falls gewünscht können die Ester auch Substituenten enthalten, die aber auf keinen Fall mit den Isocyanatgruppen reaktiv sein dürfen wie die reaktiven Hydroxylgruppen. Als Fettsäuren sind beispielsweise Capron-, Capryl-, Pelargon-, Caprin-, Laurin-, Myristin-, Palmitin-, Stearin-, Arachin-, Behen-, Laurolein-, Myristolein-, Palmitolein-, 01-, Linol- und/oder Linolensäure geeignet sowie ihre technischen Mischungen und Fraktionen, wie sie bei der Spaltung und Destillation von natürlichen Fetten und Ölen entstehen. Besonders bevorzugt werden technische Mischungen und Fraktionen dieser Fettsäuren, die aus Kokos-, Palmkern-, Erdnuß-, Sojabohnen-, Sonnenblumen-, Lein-, erucasäurearmes und erucasäurereiches Rüböl und/oder auch Schmalz- und Rindertalg zugänglich sind.

Als aliphatische Alkohole, die mit den obengenannten Fettsäuren verestert sind, sind die aus dem Stand der Technik bekannten verzweigten und unverzweigten Alkohole wie Methanol, Ethanol, Propanol, Butanol, Pentanol, 2-Ethylhexanol, Guerbetalkohole sowie ungesättigte Vertreter oder aber auch die Fettalkohole, die sich durch Hydrierung der obengenannten Fettsäuren ableiten, geeignet. Besonders bevorzugt im Sinne der Erfindung werden Methylfettsäureester.

Die Ester sollen bei Raumtemperatur flüssig sein, d. h. unter Normaldruck bei etwa 25 °C bis 20 °C.

Die Ester werden den Isocyanten zugesetzt, vorzugsweise den höherviskosen Di-, Tri- und/oder Tetraisocyanaten und/oder Prepolymere mit 2 bis 4 Isocyanaten, die sich von den Di-, Tri- und/oder Tetraisocyanaten ableiten. Von den Di- und höherfunktionellen Tetraisocyanaten werden bevorzugt die aromatischen Vertreter hiervon und insbesondere die aromatischen Diisocyanate wie 1,5-Naphthylen-, 4,4'-Diphenylmethan-, 4,4-Diphenyldimethylmethan-, Di- und Tetraalkyldiphenylmethan-, 4,4'-Dibenzyldiisocyanat und die Isomeren des Toluylendiisocyanats. Geeignete Vertreter für Tri- und Tetraisocyanate sind die handelsüblichen Produkte wie Desmodur^{R} VKS, Fa. Bayer; Voronate ^{R} 229, Dow Chemical und/oder Basonate^{R} A 270, Fa. BASF.

Bei der Gruppe der Isocyanatgruppen tragenden Prepolymere kommen solche in Betracht, die durch Umsetzung der obigen Isocyanate mit Polyolen wie Polyetherpolyole, Polyesterpolyole, Polyalkylendiole und/oder Polyacetale erhalten werden. Die genannten Polyole und ihre Herstellung sind aus dem Stand der Technik bekannt.

So können beispielsweise Polyesterpolyole durch Reaktion von Dicarbonsäuren mit Triolen oder einem Überschuß an Diolen und/oder Triolen sowie durch Ringöffnung von epoxydierten (Fett-)Estern mit Alkoholen hergestellt werden. Auch Polycaprolactondiole, herstellbar aus E-Caprolacton und Diolen, sind als Polyesterpolyole geeignet. Im Rahmen der Erfindung werden Polyesterpolyole bevorzugt aus niedermolekularen Dicarbonsäuren wie Adipinsäure, Isophthalsäure, Terephthalsäure und Phthalsäure mit einem Überschuß an Diolen mit 2 bis 12 Kohlenstoffatomen, Trimethylolpropan und/oder Glycerin. Als Polyacetale seien beispielsweise die Polykondensationsprodukte aus Formaldehyd und Diolen und/oder Polyolen in Gegenwart saurer Katalysatoren genannt. Polyalkylendiole wie beispielsweise Polybutadiendiol sind käufliche Produkte, die in verschiedenen Molekulargewichten erhältlich sind. Polyetherpolyole können beispielsweise durch Co- oder Blockpolymerisation von Alkylenoxiden wie Ethylenoixd, Propylenoxid und Butylenoxid oder durch Umsetzung von Polyalkylenglykolen mit 2 oder 3 funktionellen Alkoholen erhalten werden. Aber auch die polymerisierten Ringöffnungsprodukte von Tetrahydrofuran mit Alkoholen sind als Polyetherpolyole geeignet.

Die Ester können erfindungsgemäß auch Isocyanuraten, den trimeren Derivaten der Isocyanursäure, wie Tris-(6-Isocyanato)-isocyanurat (Tolonate HDT^{R}, der Fa. Rhöne-Poulenc) zugesetzt werden.

Die erfindungsgemäß verwendeten Ester reduzieren bereits in geringen Zugabemengen die Viskosität der Isocyanate und/oder Isocyanurate drastisch und vor allem auch deutlich mehr als durch einfache Verdünnungseffekte zu erwarten wäre. Von weiterem Vorteil ist, daß die Viskosität der verdünnten Isocyanate und/oder Isocyanurate auch bei längerer Lagerung gar nicht oder nur unwesentlich ansteigt, so daß einmal mit den Estern verdünnte Isocyanate oder Isocyanurate gut gelagert werden können, ohne daß ständig der Ester nachdosiert werden muß. Schließlich sind die verwendeten Ester auch inert gegenüber den Isocyanaten oder Isocyanuraten, so daß keine unerwünschten Nebenreaktionen eintreten.

Um die gewünschte Viskositätserniedrigung zu erreichen, ist es zweckmäßig, die Ester im Verhältnis zu den Isocyanaten/Isocyanuraten in Gewichtsmengen von 5:95 bis 95:5 zu verwenden. Je nach gewünschter Viskosität der Isocyanate/Isocyanurate kann der Fachmann problemlos die notwendige Menge an jeweiligen Estern selber finden.

Ein weiterer Gegenstand der vorliegenden Erfindung ist eine Verfahren zur Erniedrigung der Viskosität von Isocyanaten und/oder Isocyanuraten, dadurch gekennzeichnet, daß den Isocyanaten bei Raumtempertur flüssige Ester von Fettsäuren mit 6 bis 22 C-Atomen mit monofunktionellen Alkoholen und/oder von Fettalkoholen mit 6 bis 22 C-Atomen mit Monocarbonsäuren zugegeben werden.

Einzelheiten sind den vorhergehenden Ausführungen zu entnehmen.

### Beispiele

### Beispiel 1

Zn 4,4'-Diphenylmethandiisocyanat, MDI, wurden unterschiedliche Gewichtsmengen eines Methylesters einer eurucasäurearmen Rapsölfettsäuremischung (Zusammensetzung in Gew.-%: 0 bis 5 Erucasäure (C₂₂), 50 bis 65 Ölsäure (C₁₈'), 15 bis 30 Linolsäure (C₁₈"), 6 bis 13 Linolensäure (C₁₈"'), 1 bis 3 Eicosensäure (C₂₀'), 1 bis 4 Palmitinsäure (C₁₆), und Spuren von Myristinsäure (C₁₄), Stearinsäure (C₁₈), Arachinsäure (C₂₀), Behensäure (C₂₂)), zugegeben. Der Lösemitteleffekt wurde bestimmt über die Viskosität der Mischungen nach Höppler bei 20 °C (DIN 53015). In Tabelle I sind die Ergebnisse zusammengefaßt.

**Tabelle I**

| **Viskosität** | | |
|---|---|---|
| Gew.-% Methylester | Gew.-% MDI | Viskosität 20 °C, Höppler in mPas |
| 0 | 100 | 370 |
| 10 | 90 | 137 |
| 20 | 80 | 72 |
| 30 | 70 | 44 |
| 40 | 60 | 29 |
| 50 | 50 | 20 |
| 60 | 40 | 15 |
| 70 | 30 | 12 |
| 80 | 20 | 9 |
| 90 | 10 | 8 |
| 100 | 0 | 6 |

Aus Tabelle 1 ist ersichtlich, daß die Viskosität des MDI bereits erheblich durch geringe Zugabe des Methylesters gesenkt werden kann.

### Beispiel 2

Zu 446 g MDI nach Beispiel 1 wurden 200 g eines Methylesters einer Sojaölfettsäuremischung (Zusammensetzung: 7 bis 10 Palmitinsäure (C₁₆), 3 bis 6 Stearinsäure (C₁₈), 0 bis 2 Arachinsäure (C₂₀), 20 bis 35 Ölsäure (C₁₈'), 40 bis 57 Linolsäure (C₁₈") und 5 bis 14 Linolensäure (C₁₈"') gegeben. Geprüft wurde die Lagerstabilität einer solchen Mischung, indem die Viskosität und der NCO-Gehalt über einen längeren Zeitraum beobachtet wurde. Der NCO-Gehalt wurde bestimmt durch Umsetzung der Mischung mit einem Überschuß an n-Butylamin und Rücktitration des nicht umgesetzten Butylamins mit Salzsäure. Über den Verbrauch an Butylamin wird auf den NCO-Gehalt geschlossen. In Tabelle 2 sind die Ergebnisse zusammengefaßt.

**Tabelle 2**

| **Lagertest** | | | |
|---|---|---|---|
| Tage | Viskosität 20 °C, Höppler in mPas | NCO Gehalt (Gew.-%) | visuelle Beurteilung |
| 0 | 40 | 21,3 | klare Lösung |
| 5 | 47 | n.b. | klare Lösung |
| 6 | n.b. | 21,3 | klare Lösung |
| 7 | 48 | n.b. | klare Lösung |
| 8 | n.b. | 21,4 | klare Lösung |
| 14 | 49 | 21,3 | klare Lösung |
| n.b. = nicht bestimmt | | | |

Erst nach 2 1/2 Monaten traten visuell zu beobachtende Trübungen der Mischung auf.

Aus Tabelle 2 ist zu entnehmen, daß der NCO-Gehalt sich auch nach 2 Wochen nicht geändert hat. Die Viskosität ist nach anfänglich leichtem Anstieg bei Lagerung gleich geblieben.

### Beispiel 3

Analog zu Beispiel 2 wurden Lagertests durchgeführt mit Mischungen von
3a) 466 g MDI und 200 g Methylester von Kokosfettsäuremischung (Zusammensetzung in Gew.-%: 6 bis g Caprylsäure (C₈), 6 bis 10 Caprinsäure (C₁₀), 44 bis 51 Laurinsäure (C₁₂), 13 bis 18 Myristinsäure (C₁₄), 8 bis 10 Palmitinsäure (C₁₆), 1 bis 3 Stearinsäure, 5,5 bis 7,5 Ölsäure).
3b) 466 g MDI und 200 g Methylester von eurucasäurearmer Rapsöfettsäuremischung (Zusammensetzung siehe Beispiel 1).
3c) 466 g MDI und 200 g Methylester der Laurinsäure.

In Tabelle 3 sind die Ergebnisse zusammengefaßt.

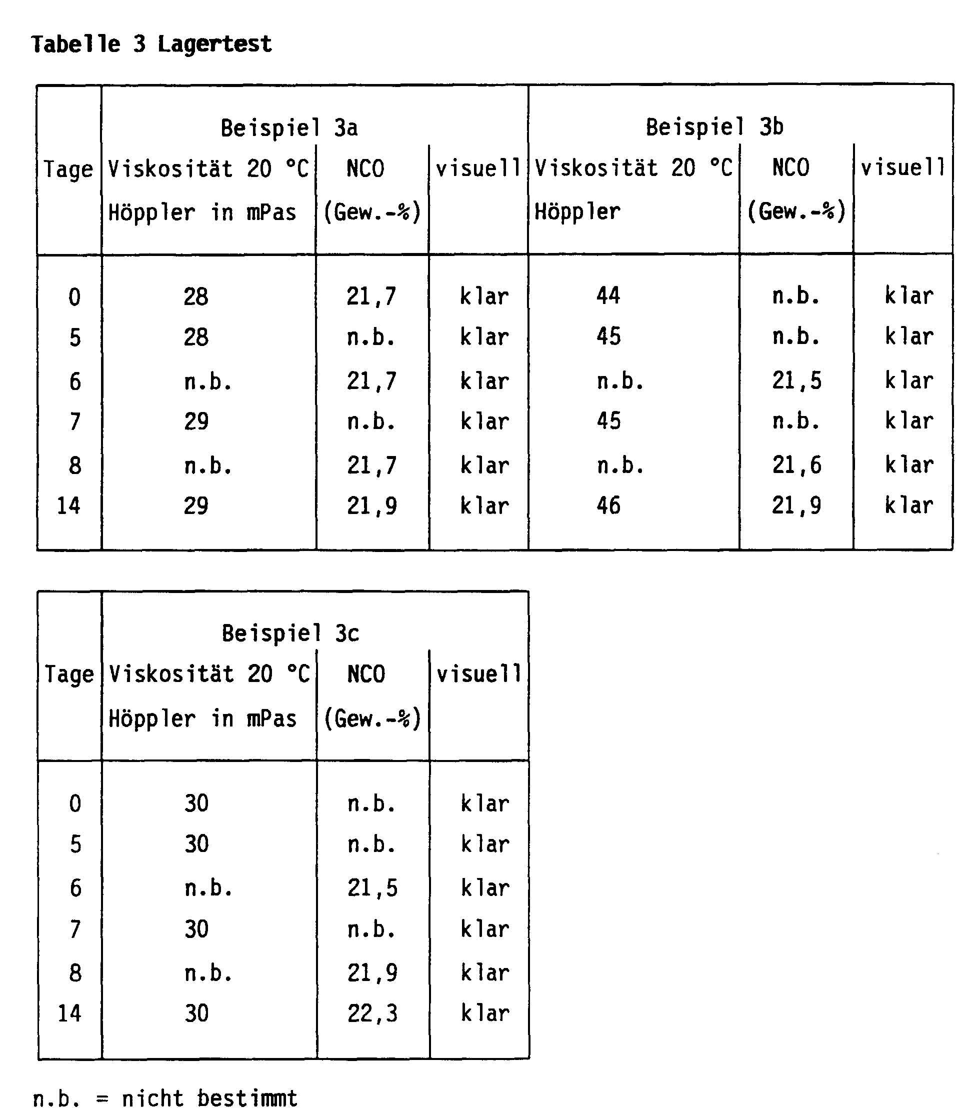

### Beispiel 4

Zu 70 g MDI (Viskosität 20 °C, nach Höppler 370 mPas) wurden
4a) 30 g Essigsäureester von technischem ungesättigtem C₁₈-Fettalkohol (Zusammensetzung in Gew.-% 0-2 C₁₂; 1-6 C₁₄; 8-18 C₁₆; 70-83 C₁₈^{,}, 0-3 C₂₀)
4b) 30 g Caprylsäureoctylester gegeben.
   Die Viskosität der klaren Mischungen 4a) und 4b) wurden nach 14 Tagen bestimmt und betrugen
4a) 37 mPas; 4b) 57 mPas.

## Patentansprüche

1. Verwendung von bei Raumtemperatur flüssigen Estern von Fettsäuren mit 6 bis 22 C-Atomen mit monofunktionellen Alkoholen als Lösungsmittel für Isocyanate und/oder Isocyanurate.

2. Verwendung nach Anspruch 1, wobei man Methylester von Fettsäuren mit 6 bis 22 C-Atomen einsetzt.

3. Verwendung nach Anspruch 1 oder 2, wobei man Isocyanate Di-, Tri- und/oder Tetraisocyanate und/oder deren Prepolymere mit 2 bis 4 Isocyanatgruppen einsetzt.

4. Verwendung nach Anspruch 3, wobei man als Isocyanate aromatische Diisocyanate einsetzt.

5. Verwendung nach einem der Ansprüche 1 bis 4, wobei man das Gewichts-Verhältnis von Estern zu Isocyanaten und/oder Isocyanuraten auf einen Wert von 5:95 bis 95:5 einstellt.

## Claims

1. The use of esters - liquid at room temperature - of C₆₋₂₂ fatty acids with monohydric alcohols as solvents for isocyanates and/or isocyanurates.

2. The use claimed in claim 1, characterized in that methyl esters of C₆₋₂₂ fatty acids are used.

3. The use claimed in claim 1 or 2, characterized in that the isocyanates are di-, tri- and/or tetraisocyanates and/or prepolymers thereof containing 2 to 4 isocyanate groups.

4. The use claimed in claim 3, characterized in that the isocyanates are aromatic diisocyanates.

5. The use claimed in any of claims 1 to 4, characterized in that the esters are used in a ratio by weight to the isocyanates and/or isocyanurates of 5:95 to 95:5.

## Revendications

1. Utilisation d'esters liquides à température ambiante d'acides gras comportant 6 à 22 atomes de C avec des alcools monofonctionnels, comme solvants pour des isocyanates et/ou des isocyanurates.

2. Utilisation selon la revendication 1, dans laquelle on utilise des esters de méthyle d'acides gras comportant 6 à 22 atomes de C.

3. Utilisation selon la revendication 1 ou 2, dans laquelle on utilise comme isocyanates, des di-, des tri- et/ou des tétraisocyanates et/ou les prépolymères de ceux-ci comportant 2 à 4 groupes isocyanate.

4. Utilisation selon la revendication 3, dans laquelle on utilise comme isocyanates, des diisocyanates aromatiques.

5. Utilisation selon une des revendications 1 à 4, dans laquelle on ajuste le rapport pondéral entre les esters et les isocyanates et/ou les isocyanurates à une valeur de 5:95 à 95:5.
